# FASCICULE DE BREVET EUROPEEN

(11) **EP 1 317 230 B1**
(45) Date de publication et mention de la délivrance du brevet: **15.08.2007**
(21) Numéro de dépôt: 01967445.6
(22) Date de dépôt: 07.09.2001
(51) Int. Cl.: A61F 5/00, A61F 2/00, A61F 2/26

(54) **DISPOSITIF DE COMMANDE DU GONFLEMENT D'UNE ENVELOPPE PROTHÉTIQUE**
VORRICHTUNG ZUR AUFBLASREGELUNG EINER PROSTHETISCHEN HÜLLE
DEVICE FOR CONTROLLING THE INFLATION OF A PROSTHETIC ENVELOPE

(30) Priorité: 11.09.2000 FR 0011819
(43) Date de publication de la demande: 11.06.2003
(73) Titulaire: Medical Innovation Developpement, 69100 Villeurbanne (FR)
(72) Inventeur: FRERING, Vincent, F-69005 Lyon (FR)
(74) Mandataire: Le Cacheux, Samuel L.R.
(86) Numéro de dépôt international: PCT/FR2001/002777
(87) Numéro de publication internationale: WO 2002/019953

(56) Documents cités:
- EP-A- 0 626 154
- WO-A-00/09049
- US-A- 4 437 457
- US-A- 5 803 897

## Description

La présente invention est relative au domaine technique des prothèses du corps humain et elle vise, plus particulièrement, celles qui possèdent, en tant qu'organes actifs, un ou plusieurs éléments à déformation réversible.

L'invention concerne, plus spécifiquement, les prothèses du type comportant une enveloppe gonflable et déformable élastiquement et qui ont pour fonction de se substituer à un organe humain défaillant ou encore de venir modifier de façon réversible une fonctionnalité d'un organe humain qui n'est pas, en tant que tel, atteint dans son intégralité et sa fonction, mais dont il apparaît indispensable, nécessaire, utile, voire préférable d'altérer au moins temporairement les capacités fonctionnelles.

Parmi les applications possibles, il convient de citer, à titres non limitatifs, les enveloppes prothétiques à fonction sphinctérienne, les enveloppes prothétiques à fonction pénienne, ainsi que les enveloppes prothétiques à fonction d'implant gastrique destinées à délimiter, en partie supérieure de l'estomac, une poche ou cavité à volume relativement restreint, communiquant avec le reste de la poche stomacale par un chenal ou un conduit calibré par l'intermédiaire d'une enveloppe prothétique ou d'un implant gastrique.

Les enveloppes mises en oeuvre aux fins ci-dessus présentent la caractéristique d'être constituées au moins pour partie d'une paroi déformable élastiquement qui peut être gonflée ou dégonflée à souhait par admission ou évacuation d'un fluide approprié, généralement liquide, plus spécifiquement du type sérum physiologique.

La fonctionnalité de telles enveloppes prothétiques dépend de la maîtrise du contrôle ou de la commande du gonflement qui peut faire intervenir une nécessité pluri-quotidienne ou épisodique selon les applications. Le document EP-A-0626154 décrit les caractéristiques techniques du préambule de la revendication 1.

Dans tous les cas cependant, il convient de pouvoir disposer de moyens techniques à même d'assurer une telle fonction avec toutes les exigences qui s'y attachent, à savoir, la discrétion, la facilité, la fiabilité et l'aisance, soit pour le sujet appareillé, soit pour le praticien chargé de contrôler la fonctionnalité de l'enveloppe prothétique qu'il a implantée.

Si les solutions avancées par la technique antérieure prennent en compte un tel objectif général, il peut être considéré que les moyens techniques proposés ne sont pas de nature à respecter intégralement les exigences rencontrées, ni à permettre une réelle, pratique, aisée et confortable implantation totale. Le besoin de résoudre ce problème général se fait sentir en particulier pour des raisons de confort dans le cadre des enveloppes prothétiques à fonction sphinctérienne ou pénienne sans qu'il faille omettre l'exigence de sécurité qu'il convient également de respecter dans le cadre d'implantation gastrique. En effet, en règle générale, une telle enveloppe prothétique, adaptée autour de la poche stomacale, est reliée à un boîtier pourvu d'une membrane auto-obturable qui peut être transpercée par une aiguille de seringue ou analogue au moyen de laquelle une injection ou un prélèvement de liquide peut être effectué pour contrôler le développement de l'enveloppe chargée d'induire un effet de striction de la poche stomacale.

Le suivi des sujets implantés implique de procéder périodiquement à des opérations de contrôle pour apprécier au moins le bon réglage de la striction induite en fonction des résultats qui sont visés pour lutter contre l'obésité alimentaire du suj et implanté.

Pour cette raison, il est fréquemment procédé à une phase de contrôle qui intervient par application de rayons X selon une méthode appropriée qui permet de délivrer au praticien une image réelle de la situation de l'implant pour décider d'une injection ou d'un prélèvement à partir du boîtier, en position sous-cutanée, en vue d'accroître ou de diminuer la striction produite.

Or, il s'avère que les praticiens se livrent à une telle activité avec une fréquence beaucoup plus importante que celle d'intervention à titre d'implantation et que, par suite, ils sont soumis à une irradiation correspondante par l'intermédiaire des appareils de radiographie ou de radioscopie mis en service.

Une telle exposition est certainement de nature à entraîner des conséquences pénalisantes pour la santé des praticiens qu'il conviendrait de pouvoir protéger au cours de l'exercice du contrôle d'implantation ou du réglage auquel ils doivent se livrer périodiquement.

C'est justement l'objet de l'invention que de répondre à ces besoins multiples attachés à la fonctionnalité des enveloppes prothétiques implantables et gonflables.

L'invention a pour objet un dispositif de commande du gonflement d'une enveloppe prothétique implantable selon la revendication 1.

L'invention a certainement aussi pour objet une prothèse implantable et gonflable mettant en oeuvre le dispositif ci-dessus.

Diverses autres caractéristiques ressortent de la description faite ci-dessus en référence aux dessins annexés qui montrent, à titre d'exemple non limitatif, une forme de réalisation de l'objet de l'invention.
La **fig. 1** est une vue schématique illustrant une forme de réalisation d'une prothèse selon l'invention dans le cadre de plusieurs applications particulières.
La **fig. 2** est une élévation schématique illustrant plus précisément l'une des applications.
**La fig. 3** est une coupe-élévation prise selon la ligne **III-III** de la **fig. 1** et montrant, à échelle différente, la structure du dispositif de commande.
Les **fig. 4** et **5** sont des coupes-élévations partielle illustrant, à plus grande échelle, des états fonctionnels du dispositif.

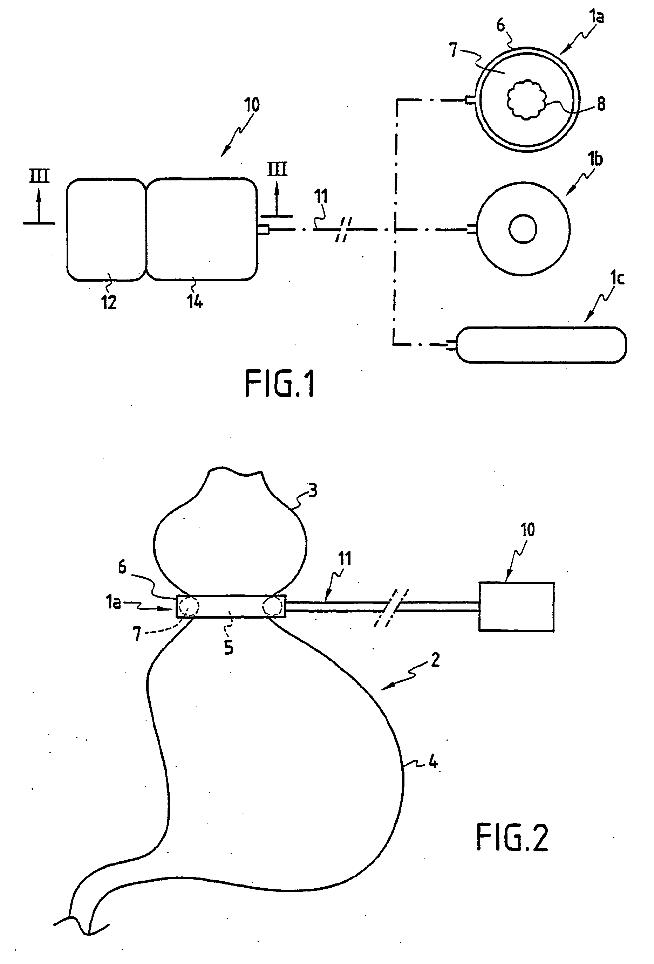

**La fig. 1** montre une prothèse gonflable **1a** ou **1b** ou **1c** implantable et réalisée sous la forme d'une enveloppe déformable élastiquement et gonflable.

Selon les applications envisagées, une telle enveloppe prothétique implantable peut présenter une conformation structurelle variable.

A titre d'exemple, l'enveloppe prothétique **1a** correspond à un implant gastrique tel qu'illustré par la **fig**. **2** et qui est destiné à être implanté en position haute sous hiatale d'un estomac **2** de manière à délimiter une poche gastrique artificielle **3** qui est en relation avec la poche gastrique inférieure **4** par l'intermédiaire d'un chenal de communication **5** dont la section de passage est contrôlée par l'implant gastrique **1a.**

Afin d'assumer la fonction décrite, l'enveloppe prothétique **1a** comprend une ceinture **6** réalisée en une matière souple mais non déformable élastiquement qui peut être avantageusement du type ouvert et posséder à ses parties terminales non représentées des moyens de liaison amovibles pour faciliter l'implantation autour de l'estomac **2**.

La ceinture **6** est associée à une enveloppe annulaire **7** qui est réalisée en une matière déformable élastiquement et qui occupe la surface périphérique intérieure de la ceinture **6** de manière que le réglage de son gonflement vienne modifier la section de passage **8** qui est responsable de la striction du chenal de communication **5**.

L'exemple en relation avec l'enveloppe prothétique **1a** n'est donné qu'à titre illustratif, étant entendu que des structures différentes et adaptées à des fonctionnalités autres pouvant être envisagées sont prises en compte.

A titre d'exemple, l'enveloppe prothétique **1b** correspond à une application sphinctérienne c'est-à-dire à une substitution d'un sphincter naturel défaillant par une enveloppe telle que **1b** dont le contrôle du gonflement permet d'ouvrir ou de fermer le sphincter naturel en fonction des exigences à respecter.

Un autre exemple est illustré par l'enveloppe prothétique **1c** qui est réalisée sous la forme d'un ballonnet gonflable, allongé, destiné à assumer une fonction érectile, par exemple en substitution d'un organe naturel défaillant.

Afin d'assurer la fonction recherchée, la prothèse comprend un dispositif 10 de commande et de contrôle du gonflement de l'enveloppe **1**.

Le dispositif **10** est destiné à offrir aux sujets implantés ou aux praticiens la possibilité de contrôler le gonflement de l'enveloppe prothétique et, à cet effet, il comprend l'ensemble des moyens suivants destinés à être eux aussi implantés généralement en couche sous-cutanée. A cet effet, le dispositif **10** décrit dans ce qui suit est relié à l'enveloppe gonflable par un tube ou conduit de transfert **11** dont la longueur peut être très variable en fonction de l'application visée.

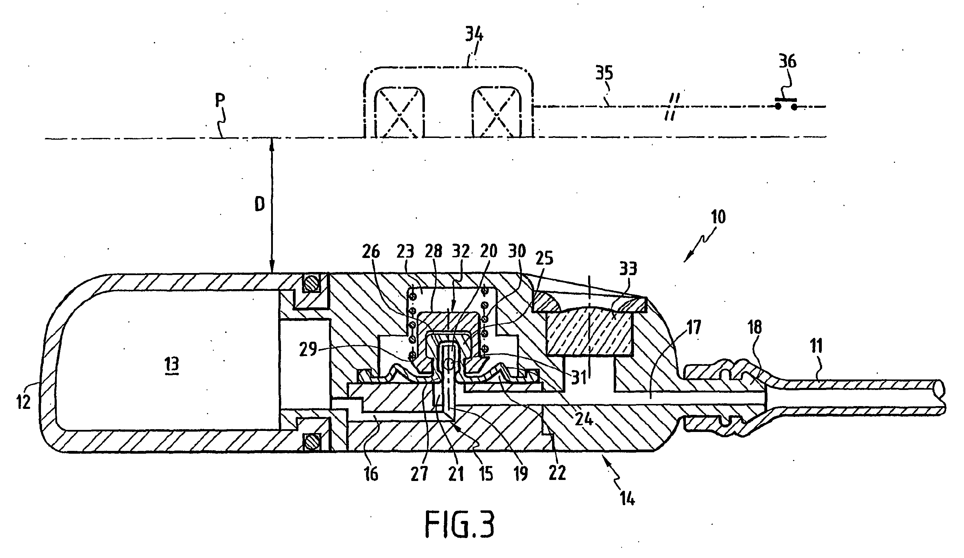

Selon la **fig. 3,** le dispositif **10** comprend une poche **12** réalisée en toute matière appropriée, déformable élastiquement, de manière à délimiter un réservoir **13** qui est apte à contenir une quantité de fluide liquide, tel que du sérum physiologique, au moins égale à la capacité d'absorption en vue de son gonflement de l'enveloppe gonflable **1a, 1b** ou **1c.** Au sens de l'invention, il est particulièrement avantageux que la poche **12** soit réalisée de manière à posséder une mémoire de forme de sa capacité de rétention maximale.

La poche **12** est associée à un boîtier de commande **14** soit directement comme cela est représenté à la **fig. 3,** soit par l'intermédiaire d'un conduit de communication mettant en relation le réservoir **13** avec la structure interne du boîtier de commande **14** qui est réalisée pour comporter intérieurement un circuit de transfert **15** possédant une branche **16** communiquant avec le réservoir **13** et une branche **17** qui s'ouvre à l'intérieur d'un embout **18** auquel est raccordé le tube ou conduit **11.**

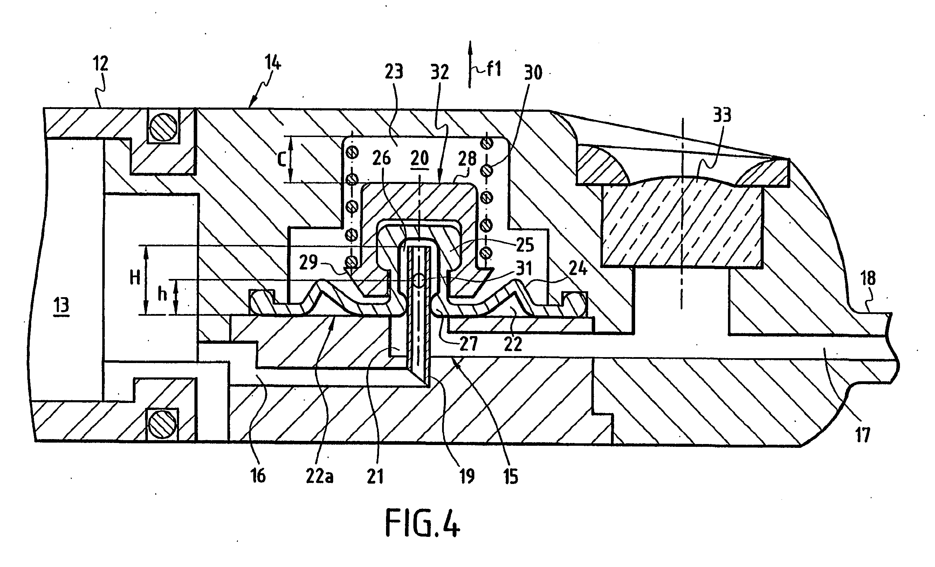

Comme cela est mis en évidence par la **fig. 4**, la première branche **16** est pourvue d'un tube **19** ouvert à son extrémité extrême **20** et qui traverse axialement une préchambre **21** présentée par la seconde branche **17** pour s'ouvrir dans une chambre **22** qui est ménagée, à partir d'une cavité **23** que présente le boîtier **14,** par l'intermédiaire d'une membrane déformable élastiquement **24.** La membrane **24** forme un mamelon **25** délimitant un puits **26** inversé dans lequel se trouve engagée la partie terminale du tube **19** sur lequel ladite membrane est enfilée par un joint d'étanchéité **27** du type à lèvre ou torique. Le mamelon **25** est coiffé d'une tête **28** réalisée en un matériau ferromagnétique et qui forme à sa base un épaulement **29** contre lequel prend appui un organe élastique de rappel **30** disposé sous contrainte préalable entre l'épaulement **29** et le fond de la cavité **23.**

L'organe élastique **30** est prévu pour maintenir l'organe sensible que constitue la membrane **24** dans une position dans laquelle la chambre **22** est réduite à son volume minimal, dans laquelle la préchambre **21** est fermée et dans laquelle au moins un trou radial **31** que présente le tube **19** s'ouvre à l'intérieur du puits **26** en étant isolé de façon étanche de la chambre **22** et de la préchambre **21.**

Par construction, l'ensemble tête **28,** mamelon **25,** membrane **24,** tube **19,** ressort **30** constitue un obturateur ou un clapet anti-retour **32** présentant la particularité d'être à ouverture commandée. En outre, ce clapet présente la caractéristique de posséder une course dite d'ouverture-fermeture **C** alors que le tube **19** s'élève, à partir du plan d'appui **22ₐ** que délimite la chambre **22** pour la membrane **24,** sur une hauteur **H** supérieure à la course **C.** Par construction toujours le trou radial **31** est pratiqué pour être situé, par rapport au plan **22ₐ** à une hauteur *h* inférieure à la course **C.**

Selon une disposition avantageuse dont l'explication est donnée ultérieurement, le boîtier **14** est réalisé de manière à comporter en outre, un septum **33** ou une membrane transperçable, auto-obturable et accessible par l'une de ses faces et qui fournit un accès par exemple pour une aiguille de seringue directement dans la seconde branche **17.**

Le boîtier **14** et la poche **12** sont destinés à être implantés en sous-cutané par exemple à faible profondeur sous la peau **P** d'un patient et en tous cas à une distance **D** permettant d'influencer la tête ferromagnétique **28** par l'intermédiaire d'une bobine **34** capable de fournir un champ magnétique d'une puissance suffisante pour vaincre l'action antagoniste de l'organe élastique **30.**

Le dispositif décrit ci-dessus permet de mettre en oeuvre le procédé suivant.

Après implantation de la prothèse et quelque soit l'application visée, il doit être compris que l'enveloppe gonflable **1a, 1b** ou **1c** se trouve dans un état de développement minimal et que le fluide liquide nécessaire à son gonflement occupe le réservoir **13** ainsi que le circuit **15** voire éventuellement le tube **11.**

La première mise en service consiste à exercer sur la poche **12** une pression de déformation par action manuelle à travers la peau **P** de manière à provoquer le transfert du fluide liquide et sa mise en pression à la fois dans la poche **12** et dans la première branche **16** de telle sorte que ce fluide liquide est introduit dans le puits **26** par le tube **19.**

L'arrivée de ce fluide liquide sous pression provoque la mise sous contrainte supplémentaire du ressort **30,** de sorte que l'organe sensible, que constitue la membrane et son mamelon, se trouve soumis à un déplacement dans le sens de la flèche **f₁** ayant pour effet de faire croître le volume de la chambre **22**.

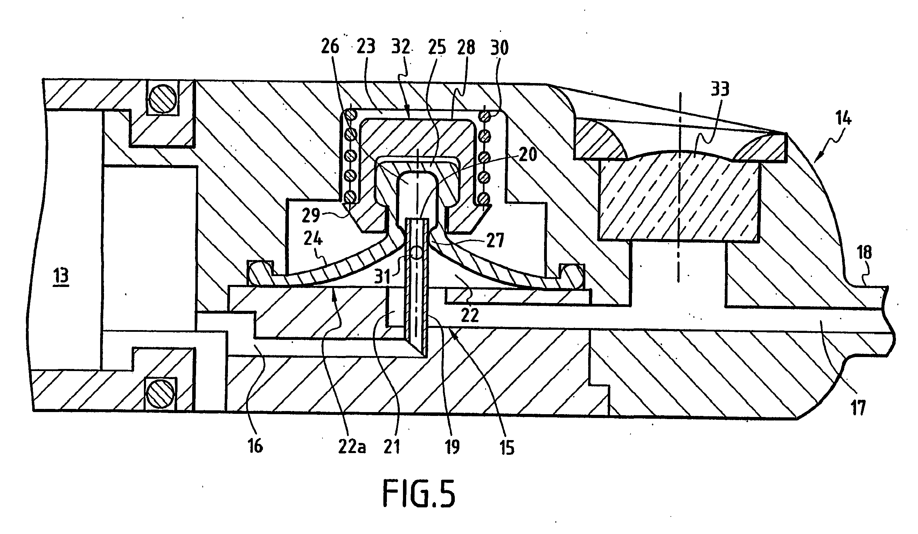

Une première conséquence en est l'ouverture de la préchambre **21** alors qu'une seconde conséquence est le coulissement de la membrane **24** sur le tube **19** jusqu'à ce que le joint d'étanchéité **27** vienne découvrir le trou radial **31** comme cela est illustré par la **fig. 5**.

L'examen de cette figure permet de constater que la première branche **16** communique par le trou **31** avec la chambre **22** qui est mise en communication avec la préchambre **21** dans laquelle s'ouvre la seconde branche **17.**

Par le moyen décrit ci-dessus, la quantité de fluide liquide contenu dans le réservoir **13** est transférée dans l'enveloppe gonflable **1a, 1b** ou **1c** jusqu'à provoquer le gonflement maximal de cette dernière.

Après gonflement, l'arrêt de l'action exercée sur la poche **12** établit un équilibre des pressions dans le circuit **15,** de sorte que le ressort **30** ramène le clapet dans la position de fermeture selon la **fig. 3** ou **4**, dans laquelle la membrane **24** isole la préchambre **21** de la chambre **22** et le joint isole la préchambre **21** du trou **31** pour interrompre toute communication entre les branches **16** et **17.**

Dans une application pouvant être qualifiée de tout ou rien, telle que l'application de l'exemple de l'enveloppe **1b** ou **1c,** la situation ainsi acquise est maintenue tant que la nécessité ne se fait pas sentir de provoquer le dégonflement de l'enveloppe **1b** ou **1c**.

Dans un tel cas, la bobine **34** est disposée à l'aplomb de la tête ferromagnétique **28** de manière à exercer par le champ qu'elle développe une action d'attraction contraire à l'action antagoniste du ressort de rappel **30.** A partir de la position représentée à la **fig. 4**, l'organe sensible est élevé dans le sens de la flèche **f₁** jusqu'au moment où la membrane **24** met en relation la chambre **22** et la préchambre **21** et dans un second temps découvre le trou **31** qui rétablit la communication entre la branche **17** et la branche **16.**

Le reflux du fluide liquide de l'enveloppe au réservoir peut alors intervenir soit naturellement par la pression que les tissus environnants exercent sur l'enveloppe, soit par la mémoire de forme de la poche **12** qui tend à réoccuper sa position initiale en favorisant le reflux du fluide liquide, soit encore par les deux actions.

Dans l'application particulière de la prothèse à un implant gastrique, la seconde phase de réglage consiste alors par impulsions successives à commander l'alimentation de la bobine **34** pour agir sur l'organe sensible du clapet que constitue l'ensemble **24, 25, 28** de manière à réadmettre par reflux des petites quantités de fluide liquide en direction du réservoir **13** pour provoquer le dégonflement progressif de l'implant gastrique jusqu'à ce que la réduction de section ou striction que ce dernier doit induire est atteinte.

Les moyens qui viennent d'être décrits dans leur structure et leur fonctionnement résolvent les problèmes rappelés ci-dessus et permettent notamment au praticien chargé de contrôler le bon fonctionnement d'un anneau gastrique, d'assurer à distance la commande en ouverture du clapet ou de l'organe sensible en disposant par exemple d'une ligne **35** d'alimentation de la bobine **34** incluant un interrupteur **36** mis à disposition du praticien en dehors de la ou des zones occupées par le patient et risquant d'être soumises à des radiations produites par les moyens techniques de visualisation par radiographie ou radioscopie ou analogue.

Outre les avantages que procure un tel dispositif, il doit être noté qu'il offre une sécurité maximale dans l'intérêt du patient en cas de dysfonctionnement ou d'immobilisation incontrôlée de l'organe sensible que constitue le clapet.

En effet, à supposer que ce clapet devienne inopérant, défectueux, ou incapable d'assumer la fonction d'ouverture-fermeture qui lui est dévolue, le praticien peut toujours intervenir manuellement et de façon directe pour admettre ou évacuer une proportion de fluide liquide en agissant par l'intermédiaire d'une seringue dont l'aiguille est amenée à perforer le septum **33** pour disposer d'un accès direct à la seconde branche **17** ainsi qu'au tube **11** conduisant au volume interne de l'enveloppe gonflable à laquelle une quantité de fluide liquide peut être ajoutée ou ôtée en fonction des besoins.

## Revendications

1. - Dispositif de commande du gonflement d'une enveloppe prothétique gonflable, comprenant :
- une poche **(12)** déformable contenant une réserve de fluide liquide apte à assurer le gonflement de ladite enveloppe **(1),**
- un boîtier **(14)** délimitant un circuit de transfert **(15)** contrôlé par un obturateur commandable **(32)** et raccordé à l'enveloppe (1) ainsi qu'à la poche (12),
**caractérisé en ce que** l'obturateur est clapet anti-retour (32) qui comprend :
- un élément mobile (24,25), d'ouverture-fermeture du circuit de transfert (15),
- un organe élastique (30) de rappel de l'élément mobile (24,25) en position de fermeture,
- et un organe sensible à caractère ferromagnétique (28) solidaire de l'élément mobile (24,25) pour commander l'ouverture de l'élément mobile sous l'effet d'un champ magnétique.

2. - Dispositif selon la revendication 1, **caractérisé en ce que** le clapet (32) est disposé dans le boîtier (14) qui comporte, dans la partie du circuit de transfert entre ledit clapet et l'enveloppe, un septum ou membrane (33) transperçable et obturable.

3. - Dispositif selon la revendication 1 ou 2, **caractérisé en ce que** le boîtier (14) est directement associé à la poche (12).

4. - Dispositif selon l'une des revendications 1 à 3 , **caractérisé en ce que** la poche (12) est déformable élastiquement et possède une mémoire de forme de sa capacité de rétention maximale.

5. - Dispositif selon l'une des revendications 1 à 4, **caractérisé en ce que** le clapet (32) est disposé sur le circuit de transfert (15) que délimite le boîtier (14) et qui comprend une première branche (16) en relation avec la poche (12), un tube (19) d'intercommunication avec la première branche et avec une chambre (22), une seconde branche (17) susceptible d'être placée en relation avec la chambre et dans cette dernière l'élément mobile (24), (25), du clapet qui est monté coulissant sur le tube pour ouvrir ou fermer la communication avec la seconde branche.

6. - Dispositif selon la revendication 5, **caractérisé en ce que** le tube d'intercommunication (19) fait saillie dans la chambre (22) qui est délimitée par l'élement mobile qui est constitué par une membrane (24) déformable élastiquement et qui forme une lèvre d'étanchéité bordant un puits (26) et enfilée sur le tube qui débouche dans le puits par son extrémité ouverte et qui possède au moins un trou radial (31) de mise en communication avec la chambre (22).

7. - Dispositif selon la revendication 6, **caractérisé en ce que** l'organe sensible est une tête ferromagnétique (28) et **en ce que** le puits (26) est délimité par un mamelon (25) que présente la membrane (24) et qui est coiffé par la tête ferromagnétique (28) associée à l'organe élastique (30) sollicitant toujours la membrane (24) dans une position de fermeture du trou radial du tube et d'interruption de communication entre la première (16) et la seconde branche (17).

8. - Dispositif selon la revendication 7, **caractérisé en ce que** la tête (28) est disposée dans une cavité (23) fermée délimitée par le boîtier et séparée de la chambre par la membrane.

9. - Dispositif selon la revendication 7 ou 8, **caractérisé en ce que** l'organe élastique est un ressort hélicoïdal et **en ce que** la tête (28) forme un épaulement (29) contre lequel prend appui le ressort hélicoïdal (30) travaillant à la compression et mis sous contrainte préalable dans la cavité.

10. - Dispositif selon la revendication 6, **caractérisé en ce que** le tube (19) présente, à compter d'un plan (22a) présenté par la chambre (22) pour l'appui de la membrane (24) en fermeture de la seconde branche (17), une hauteur H supérieure à la course C du clapet.

11. - Dispositif selon la revendication 6 ou 10, **caractérisé en ce que** le tube (19) présente au moins un trou radial (31) qui est situé à une hauteur h du plan d'appui (22a) inférieure à la course C du clapet.

12. - Prothèse implantable composée d'une enveloppe gonflable et d'un dispositif de commande de gonflement selon l'une des revendications 1 à 11.

## Claims

1. Device for controlling the inflation of an inflatable prosthetic envelope comprising:
- a deformable pouch (12) containing a liquid fluid reserve capable of inflating said envelope (1),
- a casing (14) delimiting a transfer circuit (15) controlled by a controllable closer (32) connected to the envelope (1) and to the pouch (12),
**characterised in that** the closer is a non-return valve (32) that comprises:
- a mobile element (24, 25) to open and close the transfer circuit (15),
- an elastic device (30) to return the mobile element (24, 25) into the closed position,
- and a sensitive ferromagnetic device (28) fixed to the mobile element (24, 25) to control opening of the mobile element under the effect of a magnetic field.

2. Device according to claim 1, **characterised in that** the valve (32) is placed in the casing (14) that comprises a septum or membrane (33) that can be perforated and closed off, in the part of the transfer circuit between said valve and the envelope.

3. Device according to claim 1 or 2, **characterised in that** the casing (14) is directly associated with the pouch (12) .

4. Device according to one of claims 1 to 3, **characterised in that** the pouch (12) is elastically deformable and has a shape memory of its maximum retention capacity.

5. Device according to one of claims 1 to 4, **characterised in that** the valve (32) is arranged on the transfer circuit (15) that delimits the casing (14) and comprises a first branch (16) related to the pouch (12), an interconnection tube (19) between the first branch and a chamber (22), a second branch (17) that can be connected with the chamber, the chamber containing the mobile element (24,25) of the valve mounted free to slide on the tube to open or close the communication with the second branch.

6. Device according to claim 5, **characterised in that** the intercommunication tube (19) projects into the chamber (22) that is delimited by the mobile element that is composed of an elastically deformable membrane (24) and that forms a lip seal surrounding a well (26) that is slid onto the tube that opens up into the well at its open end and that is provided with at least one radial hole (31) making a communication with the chamber (22).

7. Device according to claim 6, **characterised in that** the sensitive device is a ferromagnetic head (28) and **in that** the well (26) is delimited by a nipple (25) on the membrane (24) and that is capped by the ferromagnetic head (28) associated with the elastic device (30), that always forces the membrane (24) into a position to close the radial hole in the tube and interrupt the communication between the first branch (16) and the second branch (17).

8. Device according to claim 7, **characterised in that** the head (28) is placed in a closed cavity (23) delimited by the casing and separated from the chamber by the membrane.

9. Device according to claim 7 or 8, **characterised in that** the elastic device is a helical spring and **in that** the head (28) forms a shoulder (29), against which the helical spring (30) previously stressed in compression in the cavity applies pressure.

10. Device according to claim 6, **characterised in that** starting from a plane (22a) included in the chamber (22) to act as support for the membrane (24) closing the second branch (17), the tube (19) has a height H greater than the travel distance C of the valve.

11. Device according to claim 6 or 10, **characterised in that** the tube (19) has at least one radial hole (31) that is located at a height h from the bearing plane (22a) less than the travel distance C of the valve.

12. Implantable prosthesis composed of an inflatable envelope and an inflation control device according to one of claims 1 to 11.

## Patentansprüche

1. Vorrichtung zur Steuerung des Aufblasens einer aufblasbaren prothetischen Hülle, umfassend:
- eine verformbare Tasche (12), die eine Flüssigkeitsreserve enthält, die das Aufblasen der Hülle (1) gewährleisten kann,
- ein Gehäuse (14), das eine Weiterleitungsschaltung (15) begrenzt, die von einem steuerbaren Verschlußmittel (32) kontrolliert wird und an die Hülle (1) sowie an die Tasche (12) angeschlossen ist,
**dadurch gekennzeichnet, daß** das Verschlußmittel eine Rückschlagklappe (32) ist, die umfaßt:
- ein bewegliches Element (24, 25) zum Öffnen und Schließen der Weiterleitungsschaltung (15),
- ein elastisches Element (30) zur Rückstellung des beweglichen Elements (24, 25) in Verschlußposition,
- und ein sensibles Element mit ferromagnetischem Charakter (28), das mit dem beweglichen Element (24, 25) verbunden ist, um das Öffnen des beweglichen Elements und der Wirkung eines Magnetfeldes zu steuern.

2. Vorrichtung nach Anspruch 1, **dadurch gekennzeichnet, daß** die Klappe (32) in dem Gehäuse (14) angeordnet ist, das in dem Teil der Weiterleitungsschaltung zwischen der Klappe und der Hülle ein Septum oder eine Membran (33), die durchstoßbar und verschließbar ist, umfaßt.

3. Vorrichtung nach Anspruch 1 oder 2, **dadurch gekennzeichnet, daß** das Gehäuse (14) direkt mit der Tasche (12) verbunden ist.

4. Vorrichtung nach einem der Ansprüche 1 bis 3, **dadurch gekennzeichnet, daß** die Tasche (12) elastisch verformbar ist und ein Formgedächtnis bezüglich seiner maximalen Aufnahmekapazität besitzt.

5. Vorrichtung nach einem der Ansprüche 1 bis 4, **dadurch gekennzeichnet, daß** die Klappe (32) auf der Weiterleitungsschaltung (15) angeordnet ist, die das Gehäuse (14) begrenzt und die einen ersten Abschnitt (16) in Verbindung mit der Tasche (12), ein Rohr (19) zur Verbindung zwischen dem ersten Abschnitt und einer Kammer (22) und einen zweiten Abschnitt (17) umfaßt, der mit der Kammer und in dieser letztgenannten mit dem beweglichen Element (24), (25) der Klappe in Verbindung gebracht werden kann, die gleitend auf dem Rohr montiert ist, um die Verbindung mit dem zweiten Abschnitt zu öffnen oder zu schließen.

6. Vorrichtung nach Anspruch 5, **dadurch gekennzeichnet, daß** das Verbindungsrohr (19) in die Kammer (22) ragt, die von dem beweglichen Element begrenzt ist, das von einer elastisch verformbaren Membran (24) gebildet ist, die eine Dichtlippe bildet, die einen Schacht (26) umrahmt und über das Rohr geschoben ist, das in den Schacht mit seinem offenen Ende mündet, und das mindestens ein Radialloch (31) zur Verbindung mit der Kammer (22) aufweist.

7. Vorrichtung nach Anspruch 6, **dadurch gekennzeichnet, daß** das sensible Element ein ferromagnetischer Kopf (28) ist und daß der Schacht (26) von einem Anschlußstück (25) begrenzt ist, das die Membran (24) aufweist und das von dem ferromagnetischen Kopf (28), der mit dem elastischen Element (30) verbunden ist, bedeckt ist, wobei die Membran (24) immer in eine Position zum Verschließen des Radialloches des Rohrs und zur Unterbrechung der Kommunikation zwischen dem ersten (16) und dem zweiten Abschnitt (17) gedrückt wird.

8. Vorrichtung nach Anspruch 7, **dadurch gekennzeichnet, daß** der Kopf (28) in einem geschlossenen Hohlraum (23) angeordnet ist, der vom Gehäuse begrenzt und von der Kammer durch die Membran getrennt ist.

9. Vorrichtung nach Anspruch 7 oder 8, **dadurch gekennzeichnet, daß** das elastische Element eine Spiralfeder ist, und daß der Kopf (28) einen Absatz (29) bildet, an dem die Spiralfeder (30) zur Anlage gelangt, die zusammengedrückt und im Hohlraum einer Vorspannung unterzogen wird.

10. Vorrichtung nach Anspruch 6, **dadurch gekennzeichnet, daß** das Rohr (19) ab einer Ebene (22a), die von der Kammer (22) für die Abstützung der Membran (24) beim Verschließen des zweiten Abschnitts (17) gebildet ist, eine größere Höhe H als der Weg C der Klappe aufweist.

11. Vorrichtung nach Anspruch 6 oder 10, **dadurch gekennzeichnet, daß** das Rohr (19) mindestens ein Radialloch (31) aufweist, das sich auf einer Höhe h der Stützebene (22a), die geringer als der Weg C der Klappe ist, befindet.

12. Implantierbare Prothese, bestehend aus einer aufblasbaren Hülle und einer Vorrichtung zur Aufblasregelung nach einem der Ansprüche 1 bis 11.
